# EUROPEAN PATENT APPLICATION

(11) **EP 1 975 550 A1**
(43) Date of publication of application: **01.10.2008**
(21) Application number: 08005868.8
(22) Date of filing: 27.03.2008
(51) Int. Cl.: G01B 9/02, A61B 3/12, A61B 3/15, A61B 5/00, G01N 21/47

(54) **Optical image measurement device**

(30) Priority: 28.03.2007 JP 2007085237
(71) Applicant: Kabushiki Kaisha TOPCON, Tokyo-to (JP)
(72) Inventor: Tsukada, Hisashi, Itabashi-ku Tokyo (JP); Okada, Hiroaki, Itabashi-ku Tokyo (JP); Nishio, Yutaka, Itabashi-ku Tokyo (JP); Yumikake, Kazuhiko, Itabashi-ku Tokyo (JP)
(74) Representative: Gagel, Roland

(57) **Abstract**

An optical image measurement device has: an interference-light generator (150) configured to generate an interference light (LC) by splitting a low-coherence light into a signal light (LS) and a reference light (LR) and superimposing the signal light having passed through an eye (E) and the reference light having passed through a reference object (174); a detector (184) configured to detect the generated interference light; and a scanner (141) configured to scan a projection position of the signal light on the eye, and the optical image measurement device is configured to form an image of the eye based on a result of detection by the detector. The optical image measurement device comprises a projector (140) configured to project fixation information for fixing the eye onto a fundus oculi (EF) of the eye when the scanner scans with the signal light.

## Description

### BACKGROUND OF THE INVENTION

### 1. Field of the Invention

The present invention relates to an optical image measurement device configured to scan an eye with a light beam and form an image of the eye by using the reflected light.

### 2. Description of the Related Art

In recent years, attention has been focused on an optical image measurement technique of forming an image showing the surface morphology or internal morphology of a measurement object by using a light beam from a laser light source or the like. Because this optical image measurement technique does not have invasiveness to human bodies unlike an X-ray CT device, it is expected to employ this technique particularly in the medical field.

Japanese Unexamined Patent Application Publication JP-A 11-325849 discloses an optical image measurement device configured in a manner that: a measuring arm scans an object by using a rotary deflection mirror (Galvano mirror); a reference mirror is disposed to a reference arm; at the outlet thereof, such an interferometer is used that the intensity of a light caused by interference of light fluxes from the measuring arm and the reference arm is analyzed by a spectrometer; and the reference arm is provided with a device gradually changing the light flux phase of the reference light in non-continuous values.

The optical image measurement device disclosed in JP-A 1 1-325849 uses a method of so-called "Fourier Domain OCT (Optical Coherence Tomography)." That is to say, the morphology of the measurement object in the depth direction (z-direction) is imaged by applying a beam of a low-coherence light to a measurement object, obtaining the spectrum intensity distribution of the reflected light, and subjecting the obtained distribution to Fourier transform.

Furthermore, the optical image measurement device described in JP-A 11-325849 is provided with a Galvano mirror scanning with a light beam (a signal light), thereby being capable of forming an image of a desired measurement region of a measurement object. Because this optical image measurement device scans with the light beam only in one direction (x-direction) orthogonal to the z-direction, a formed image is a 2-dimensional tomographic image in the depth direction (z-direction) along the scanning direction of the light beam (the x-direction).

Further, Japanese Unexamined Patent Application Publication JP-A 2002-139421 discloses a technique of scanning with a signal light in both the horizontal and vertical directions to thereby form a plurality of 2-dimensional tomographic images in the horizontal direction and, based on these plurality of tomographic images, acquiring and imaging 3-dimensional tomographic information of a measurement range. A method for 3-dimensional imaging is, for example, a method of arranging and displaying a plurality of tomographic images in the vertical direction (referred to as stack data or the like), and a method of forming a 3-dimensional image by subjecting a plurality of tomographic images to a rendering process.

Further, Japanese Unexamined Patent Application Publication JP-A 2003-000543 discloses a configuration of using such an optical image measurement device in the ophthalmic field.

In a case where a conventional optical image measurement device is used in the ophthalmic field, a problem as described below may occur. In a measurement with an optical image measurement device, a low-coherence light having a central wavelength of a near-infrared region is used. However, because this low-coherence light also contains a visible light component, a subject tends to follow a scan trajectory, and an accurate image cannot be acquired in some cases. For example, in the case of scan with a signal light as in JP-A 2002-139421, there is a case where an eye moves in the vertical direction because a linear image moving in the vertical direction is viewed.

### SUMMARY OF THE INVENTION

The present invention was made to solve such a problem, and an object of the present invention is to provide a technique for, at the time of measurement using an optical image measurement device of a type of scanning an eye with a light beam, preventing the eye from following a scan trajectory.

In order to achieve the aforementioned object, in an aspect of the present invention, an optical image measurement device has: an interference-light generator configured to generate an interference light by splitting a low-coherence light into a signal light and a reference light and superimposing the signal light having passed through an eye and the reference light having passed through a reference object; a detector configured to detect the generated interference light; and a scanner configured to scan a projection position of the signal light on the eye, and the optical image measurement device is configured to form an image of the eye based on a result of detection by the detector. The optical image measurement device comprises a projector configured to project fixation information for fixing the eye onto a fundus oculi of the eye when the scanner scans with the signal light.

According to the present invention, at the time of measurement using an optical image measurement device of a type of scanning an eye with a light beam, it is possible to project fixation information onto a fundus oculi and fix the eye, when a scanner scans with the signal light (i.e., when the measurement is performed). Therefore, it is possible to prevent the eye from following a scan trajectory.

### BRIEF DESCRIPTION OF THE DRAWINGS

Fig. 1 is a schematic configuration diagram showing an example of the entire configuration in a preferred embodiment of a fundus oculi observation device functioning as the optical image measurement device according to the present invention.
Fig. 2 is a schematic configuration diagram showing an example of the configuration of a scan unit installed in a retinal camera unit in the preferred embodiment of the fundus oculi observation device functioning as the optical image measurement device according to the present invention.
Fig. 3 is a schematic configuration diagram showing an example of the configuration of an OCT unit in the preferred embodiment of the fundus oculi observation device functioning as the optical image measurement device according to the present invention.
Fig. 4 is a schematic block diagram showing an example of the hardware configuration of an arithmetic and control unit in the preferred embodiment of the fundus oculi observation device functioning as the optical image measurement device according to the present invention.
Fig. 5 is a schematic block diagram showing an example of the configuration of a control system in the preferred embodiment of the fundus oculi observation device functioning as the optical image measurement device according to the present invention.
Figs. 6A and 6B are schematic views showing an example of a scanning pattern of a signal light in the preferred embodiment of the fundus oculi observation device functioning as the optical image measurement device according to the present invention. Fig. 6A shows an example of the scanning pattern of the signal light when a fundus oculi is seen from the incident side of the signal light into an eye. Fig. 6B shows an example of an arrangement pattern of scanning points on each scanning line.
Fig. 7 is a schematic view showing an example of the scanning pattern of the signal light and a pattern of a tomographic image formed along each scanning line in the preferred embodiment of the fundus oculi observation device functioning as the optical image measurement device according to the present invention.
Fig. 8 is a schematic view showing an example of the scanning pattern of the signal light in the preferred embodiment of the fundus oculi observation device functioning as the optical image measurement device according to the present invention.
Fig. 9 is a flowchart showing an example of a usage pattern in the preferred embodiment of the fundus oculi observation device functioning as the optical image measurement device according to the present invention.
Fig. 10 is a flowchart showing an example of the usage pattern in the preferred embodiment of the fundus oculi observation device functioning as the optical image measurement device according to the present invention.

### DETAILED DESCRIPTION OF THE PREFERRED EMBODIMENT

An example of a preferred embodiment of the optical image measurement device according to the present invention will be described in detail referring to the drawings.

The optical image measurement device according to the present invention is used in the ophthalmic field. To be specific, the present invention relates to an optical image measurement device of a type of scanning an eye with a light beam, and is configured to prevent the eye from following a scan trajectory by modifying information viewed by a subject at the time of scan with the light beam.

### [DEVICE CONFIGURATION]

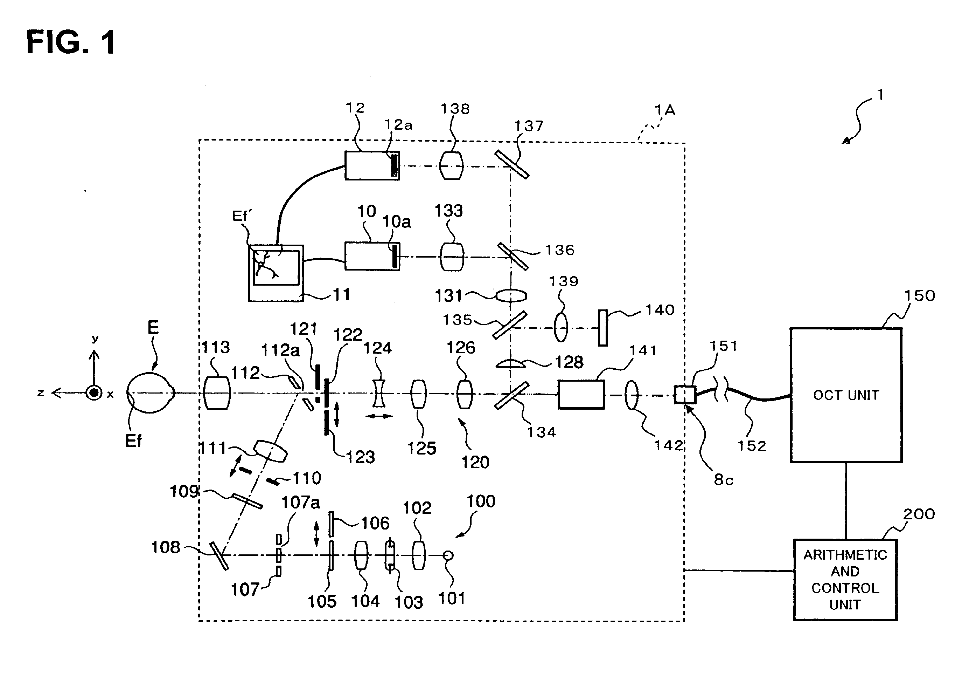

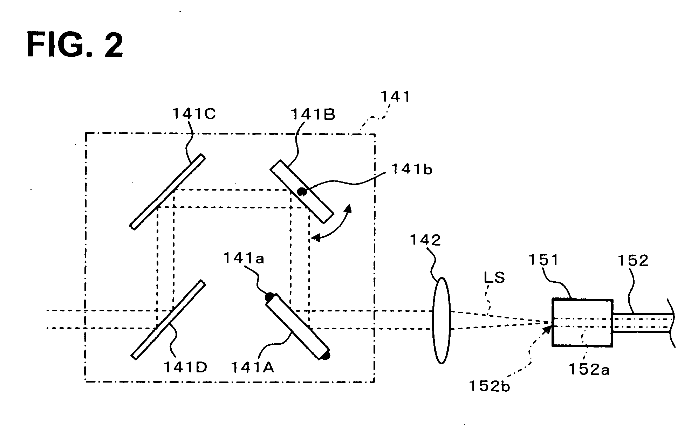

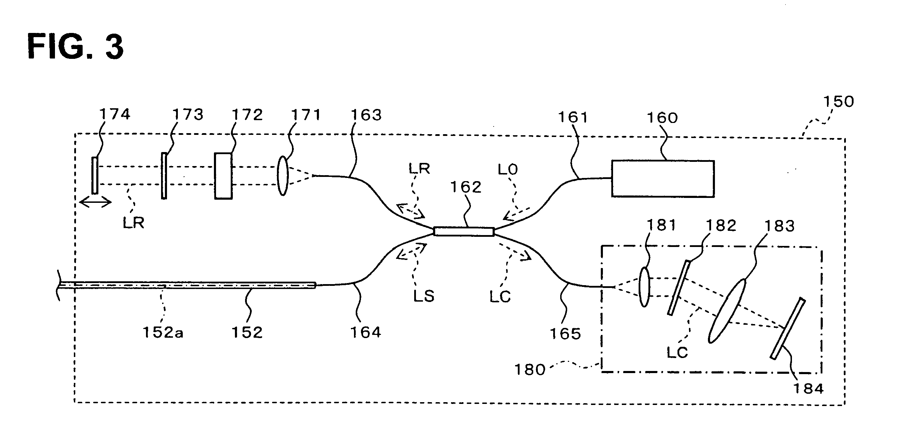

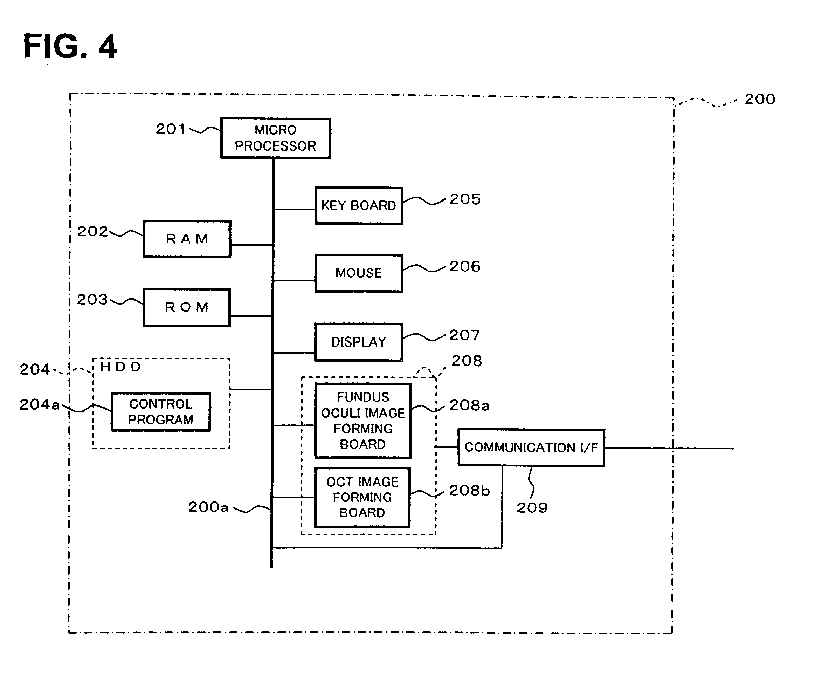

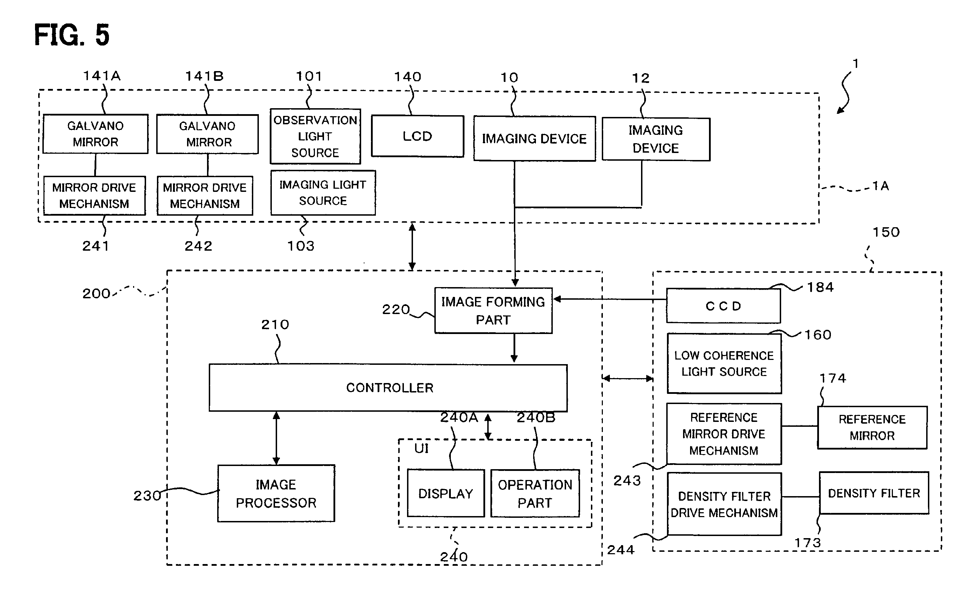

First, referring to Figs. 1 to 5, the configuration in an embodiment of the optical image measurement device according to the present invention will be described. Fig. 1 shows an example of the entire configuration of a fundus oculi observation device 1 having a function as the optical image measurement device according to the present invention. Fig. 2 shows an example of the configuration of a scan unit 141 in a retinal camera unit 1 A. Fig. 3 shows an example of the configuration of an OCT unit 150. Fig. 4 shows an example of the hardware configuration of an arithmetic and control unit 200. Fig. 5 shows an example of the configuration of a control system of the fundus oculi observation device 1.

### [ENTIRE CONFIGURATION]

The fundus oculi observation device 1 comprises the retinal camera unit 1 A, the OCT unit 150 and the arithmetic and control unit 200 as shown in Fig.1. The retinal camera unit 1 A has almost the same optical system as the conventional retinal camera capturing a 2-diensional image of the fundus oculi surface. The OCT unit 150 houses an optical system functioning as the optical image measurement device. The arithmetic and control unit 200 is equipped with a computer executing various kinds of arithmetic processes, control processes and so on.

To the OCT unit 150, one end of a connection line 152 is attached. A connector part 151 connecting the connection line 152 to the retinal camera unit 1 A is attached to the other end of the connection line 152. A conductive optical fiber runs through inside the connection line 152. Thus, the OCT unit 150 and the retinal camera unit 1A are optically connected via the connection line 152.

### [Configuration of Retinal Camera Unit]

The retinal camera unit 1A is used for forming a 2-dimensional image of the surface of a fundus oculi of an eye, based on optically acquired data (data detected by imaging devices 10 and 12). Here, the 2-dimensional image of the surface of the fundus oculi refers to a color image or monochrome image of the surface of the fundus oculi, a fluorescent image (a fluorescein angiography image, an indocyanine green fluorescent image, etc.), and the like. As well as the conventional retinal camera, the retinal camera unit 1A is provided with an illumination optical system 100 that illuminates a fundus oculi Ef, and an imaging optical system 120 that guides the fundus oculi reflection light of the illumination light to the imaging device 10.

Although the details will be described later, the imaging device 10 in the imaging optical system 120 detects an illumination light having a wavelength of a near-infrared region. Moreover, the imaging optical system 120 is further provided with the imaging device 12 detecting an illumination light having a wavelength of a visible region. Moreover, the imaging optical system 120 guides a signal light coming from the OCT unit 150 to the fundus oculi Ef, and guides the signal light having passed through the fundus oculi Ef to the OCT unit 150.

The illumination optical system 100 includes: an observation light source 101; a condenser lens 102; an imaging light source 103; a condenser lens 104; exciter filters 105 and 106; a ring transparent plate 107; a mirror 108; an LCD (Liquid Crystal Display) 109; an illumination diaphragm 110; a relay lens 111; an aperture mirror 112; and an objective lens 113.

The observation light source 101 outputs an illumination light having a wavelength of a visible region included in a range of about 400 nm to 700 nm, for example. Moreover, the imaging light source 103 outputs an illumination light having a wavelength of a near-infrared region included in a range of about 700 nm to 800 nm, for example. The near-infrared light outputted from the imaging light source 103 is set so as to have a shorter wavelength than the light used by the OCT unit 150 (described later).

Further, the imaging optical system 120 includes: the objective lens 113; the aperture mirror 112 (an aperture 112a thereof); an imaging diaphragm 121; barrier filters 122 and 123; a variable magnifying lens 124; a relay lens 125; an imaging lens 126; a dichroic mirror 134; a field lens 128; a half mirror 135; a relay lens 131; a dichroic mirror 136; an imaging lens 133; the imaging device 10 (image pick-up element l0a); a reflection mirror 137; an imaging lens 138; the imaging device 12 (image pick-up element 12a); a lens 139; and an LCD 140.

The dichroic mirror 134 is configured to reflect the fundus oculi reflection light (having a wavelength included in the range of about 400 nm to 800 nm) of the illumination light from the illumination optical system 100, and to transmit a signal light LS (having a wavelength included in the range of, for example, about 800 nm to 900 nm; described later) from the OCT unit 150.

Further, the dichroic mirror 136 is configured to transmit the illumination light having a wavelength of the visible region from the illumination optical system 100 (a visible light having a wavelength of about 400 nm to 700 nm outputted from the observation light source 101), and to reflect the illumination light having a wavelength of the near-infrared region (a near-infrared light having a wavelength of about 700 nm to 800 nm outputted from the imaging light source 103).

On the LCD 140, a fixation target (an internal fixation target) or the like for fixing the eye E is displayed. A light from the LCD 140 is reflected by the half mirror 135 after being converged by the lens 139, and is reflected by the dichroic mirror 136 after having passed through the field lens 128. Furthermore, this light passes through the imaging lens 126, the relay lens 125, the variable magnifying lens 124, the aperture mirror 112 (the aperture 112a thereof), the objective lens 113 and so on, and enters into the eye E. Consequently, the internal fixation target is projected on the fundus oculi Ef of the eye E.

The LCD 140 is an example of the "fixation-target projector" in the present invention. The fixation-target projector may be an outer fixation target projector configured to project a fixation target from the outside of the casing of the retinal camera unit 1 A.

Further, the LCD 140 displays specific visible information. The visible information is for preventing the eye E from following a scan trajectory of the signal light LS during measurement of a tomographic image of the fundus oculi Ef.

The characteristic of the visible information will be described. The visible information, for the purpose of itself, is desired to be easier to view than the scan trajectory of the signal light LS viewed during measurement of an image. In a case where visible information easier to view than the trajectory of the signal light LS is used, a possibility that a subject views the visible information during measurement of an image is high, and consequently, the subject is prevented from following the trajectory of the signal light LS.

A method for realizing the "easiness to view" of the visible information is, for example: (1) presenting visible information brighter than the trajectory of the signal light LS; (2) presenting visible information in a color that is different from the trajectory of the signal light LS, preferably, in a color that is more outstanding than that of the trajectory; (3) presenting static visible information acting so as to fix the eye E on one point; (4) presenting dynamic visible information whose form such as shape and position changes independently from the trajectory of the signal light LS; and (5) presenting dynamic visible information whose form changes accompanying the trajectory of the signal light LS.

A specific example of the visible information will be described. In the method (1), for example, the brightness of the trajectory of the signal light LS is previously measured, and visible information brighter than the measurement result is displayed on the LCD 140. Such visible information is determined in the following manner, for example. First, a photodetector is set in front of the objective lens 113. Next, a low-coherence light source 160 (described later) is turned on, an outputted low-coherence light is detected by the photodetector, and the amount of the received light (the reference light amount) is recorded. Subsequently, the visible information is displayed on the LCD 140, a light thereof is detected by the photodetector, and the amount of the received light is acquired. Then, it is determined whether the amount of the received light is larger than the reference light amount. The brightness of the visible information displayed on the LCD 140 is regulated so that the amount of the received light from the visible information becomes larger than the reference light amount. At this moment, it is desirable to set the brightness of the visible information displayed on the LCD 140 so that the amount of the received light from the visible information becomes sufficiently larger than the reference light amount. In consideration of a problem of miosis of an eye, for an eye having a small pupil, it is desirable to employ a way of emphasizing the visible information by not brightness but blinking etc.

An example of the method (2) will be described. The low-coherence light is a broadband light mainly composed of a near-infrared region as described later. Therefore, the trajectory of the signal light LS is viewed in a red color within a black-colored background. It is possible to set the color of the visible information to a color that is more outstanding than the red color within the black-colored background, in consideration of, for example, hue, saturation and brightness. Also, in the method (2), it is desirable to present the visible information brighter than the trajectory of the signal light LS.

An example of the method (3) will be described. This visible information is static information that acts so as to fix the eye E on one point. "Static" means not changing the shape, position or any other form, i.e., remaining in a constant form at all times while the information is being presented. An example of such visible information is a target that is composed of an image or the like representing the depth and acts so as to make a subject stare at the deepest part in the depth. Moreover, it is also possible to use visible information composed of a plurality of circles arranged concentrically. In the method using this visible information, the eye E is fixed on the center of the concentric circles. In addition, it is also possible to use a spirally-shaped target.

An example of the method (4) will be described. This visible information is dynamic information whose form such as shape and position changes independently from the trajectory of the signal light LS. "Dynamic" means that the shape, position or the like changes. As such visible information, for example, it is possible to use a target whose position and form change so as guide the viewpoint of the eye E in a specific direction. Here, the specific direction is, for example, a direction in which the viewpoint of the eye E is moved in order to acquire an image of a target region within the fundus oculi Ef. This direction is the same as a fixation direction by an internal fixation target or the like before measurement, for example. A presentation example of such visible information is a method of presenting a target so as to surround a position of the abovementioned specific direction. This fixation target may be of any shape, such as a circular shape and a rectangular shape. Further, as another presentation example of the visible information, it is also possible to present a target that converges into the abovementioned specific direction. This target is, for example, a concentrically-shaped target whose diameter changes to get smaller and smaller. Further, it is also possible to use a rotating spirally-shaped target. Moreover, it is also possible to use a blinking target.

An example of the method (5) will be described. This visible information is dynamic information whose form changes accompanying the trajectory of the signal light LS. As such visible information, it is possible to use one shown below. For example, in JP-A 2002-139421 mentioned before, the trajectory of the signal light LS is viewed in a manner that a laterally-extending line moves in the longitudinal direction, or in a manner that a longitudinally-extending line moves in the lateral direction. In this case, it is possible to present visible information with the same shape as the trajectory (that is, a line extending in the longitudinal or lateral direction) while moving it in the opposite direction to the trajectory. At this moment, it is desirable to move the visible information so as to be always positioned symmetrically with the trajectory with respect to the visual field center of the eye E (the fixation position by an internal fixation target or the like).

The image pick-up element l0a is an image pick-up element such as a CCD (Charge Coupled Device) and a CMOS (Complementary Metal Oxide Semiconductor) installed in the imaging device 10 such as a TV camera, and detects particularly a light having a wavelength of the near-infrared region. In other words, the imaging device 10 is an infrared TV camera that detects a near-infrared light. The imaging device 10 outputs video signals as a result of detection of the near-infrared light.

A touch panel monitor 11 displays a 2-dimensional image of the surface of the fundus oculi Ef (the fundus oculi image Ef'), based on these video signals. Moreover, these video signals are sent to the arithmetic and control unit 200, and a fundus oculi image is displayed on the display (described later).

For imaging a fundus oculi by the imaging device 10, for example, an illumination light outputted from the imaging light source 103 of the illumination optical system 100 and having a wavelength of the near-infrared region is used.

On the other hand, the image pick-up element 12a is an image pick-up element such as a CCD and a CMOS installed in the imaging device 12 such as a TV camera, and particularly detects a light having a wavelength of the visible region. That is, the imaging device 12 is a TV camera detecting a visible light. The imaging device 12 outputs video signals as a result of detection of the visible light.

The touch panel monitor 11 displays a 2-dimensional image of the surface of the fundus oculi Ef (the fundus oculi image Ef'), based on these video signals. Moreover, these video signals are sent to the arithmetic and control unit 200, and a fundus oculi image is displayed on the display (described later).

For imaging a fundus oculi by the imaging device 12, for example, an illumination light outputted from the observation light source 101 of the illumination optical system 100 and having a wavelength of the visible region is used.

The retinal camera unit 1A is provided with a scan unit 141 and a lens 142. The scan unit 141 includes a component for scanning a projection position on the fundus oculi Ef with a light outputted from the OCT unit 150 (the signal light LS; described later). The scan unit 141 is an example of the "scanner" of the present invention.

The lens 142 makes the signal light LS guided from the OCT unit 150 through the connection line 152 enter into the scan unit 141 in the form of a parallel light flux. Moreover, the lens 142 converges the fundus oculi reflection light of the signal light LS having passed through the scan unit 141.

Fig. 2 shows an example of the configuration of the scan unit 141. The scan unit 141 includes Galvano mirrors 141A and 141B, and reflection mirrors 141C and 141D.

The Galvano mirrors 141A and 141B are reflection mirrors disposed so as to be rotatable about rotary shafts 141a and 141b, respectively. The Galvano mirrors 141 A and 141 B are rotated about the rotary shafts 141a and 141b, respectively, by a drive mechanism described later (mirror drive mechanisms 241 and 242 shown in Fig. 5). Consequently, the reflection faces (faces reflecting the signal light LS) of the Galvano mirrors 141A and 141B are turned around, respectively.

The rotary shafts 141a and 141b are arranged orthogonally to each other. In Fig. 2, the rotary shaft 141 a of the Galvano mirror 141A is arranged in parallel to the paper face. On the other hand, the rotary shaft 141b of the Galvano mirror 141B is arranged in the orthogonal direction to the paper face.

That is to say, the Galvano mirror 141B is formed so as to be rotatable in the directions indicated by an arrow pointing in both directions in Fig. 2, whereas the Galvano mirror 141A is formed so as to be rotatable in the directions orthogonal to the arrow pointing in both the directions. Consequently, the Galvano mirrors 141A and 141B act so as to turn directions of reflecting the signal light LS into directions orthogonal to each other. As seen from Figs. 1 and 2, scan with the signal light LS is performed in the x-direction when the Galvano mirror 141A is rotated, and scan with the signal light LS is performed in the y-direction when the Galvano mirror 141 B is rotated.

The signal lights LS reflected by the Galvano mirrors 141 A and 141B are reflected by reflection mirrors 141C and 141D, thereby traveling in the same direction as having entered into the Galvano mirror 141A.

An end face 152b of the optical fiber 152a inside the connection line 152 is arranged facing the lens 142. The signal light LS emitted from the end face 152b travels expanding its beam diameter toward the lens 142, and is converged to a parallel light flux by the lens 142. On the contrary, the signal light LS having passed through the fundus oculi Ef is converged toward the end face 152b by the lens 142, and enters into the optical fiber 152a.

### [Configuration of OCT Unit]

Next, the configuration of the OCT unit 150 will be described referring to Fig. 3. The OCT unit 150 is a device for forming a tomographic image of a fundus oculi based on optically obtained data (data detected by a CCD 184 described later).

The OCT unit 150 has almost the same optical system as the conventional optical image measurement device. That is to say, the OCT unit 150 splits a low-coherence light into a reference light and a signal light and superimposes the signal light having passed through an eye with the reference light having passed through a reference object, thereby generating and detecting an interference light. The result of this detection (a detection signal) is inputted to the arithmetic and control unit 200. The arithmetic and control unit 200 forms a tomographic image of the eye by analyzing the detection signal.

A low-coherence light source 160 is composed of a broadband light source, such as a super luminescent diode (SLD) and a light emitting diode (LED), which outputs a low-coherence light L0. The low-coherence light LO is, for example, a light including a light with a wavelength of the near-infrared region and having a temporal coherence length of approximately several tens of micrometers.

The low-coherence light LO has a longer wavelength than the illumination light of the retinal camera unit 1A (wavelength of about 400 nm to 800 nm), for example, a wavelength included in a range of about 800 nm to 900 nm.

The low-coherence light LO outputted from the low-coherence light source 160 is guided to an optical coupler 162 through an optical fiber 161. The optical fiber 161 is composed of, for example, a single mode fiber or a PM (Polarization maintaining) fiber. The optical coupler 162 splits this low-coherence light L0 into a reference light LR and the signal light LS.

Although the optical coupler 162 acts as both a part (splitter) for splitting a light and a part (coupler) for superimposing lights, it will be herein referred to as an "optical coupler" idiomatically.

The reference light LR generated by the optical coupler 162 is guided by an optical fiber 163 composed of a single mode fiber or the like, and emitted from the end face of the fiber. Furthermore, the reference light LR is converged to a parallel light flux by a collimator lens 171, passed through a glass block 172 and a density filter 173, and reflected by a reference mirror 174. The reference mirror 174 is an example of the "reference object" of the invention.

The reference light LR reflected by the reference mirror 174 is passed through the density filter 173 and the glass block 172, converged to the fiber end face of the optical fiber 163 by the collimator lens 171 again, and guided to the optical coupler 162 through the optical fiber 163.

Here, the glass block 172 and the density filter 173 act as a delaying part for matching the optical path lengths (optical distances) of the reference light LR and the signal light LS, and also as a dispersion compensation part for matching the dispersion characteristics of the reference light LR and the signal light LS.

Further, the density filter 173 also acts as a dark filter that reduces the amount of the reference light, and is composed of a rotating ND (neutral density) filter, for example. The density filter 173 acts so as to change the reduction amount of the reference light LR by being rotary driven by a drive mechanism including a drive unit such as a motor (a density filter drive mechanism 244 described later; refer to Fig. 5). Consequently, it is possible to change the amount of the reference light LR contributing to generation of an interference light LC.

Further, the reference mirror 174 is configured to move in the traveling direction of the reference light LR (the direction of the arrow pointing both sides shown in Fig. 3). With this, it is possible to ensure the optical path length of the reference light LR according to the axial length of the eye E, the working distance (the distance between the objective lens 113 and the eye E), etc. Moreover, it is possible to capture an image of any depth position of the fundus oculi Ef, by moving the reference mirror 174. The reference mirror 174 is moved by a drive mechanism (a reference mirror drive mechanism 243 described later; refer to Fig. 5) including a driver such as a motor.

On the other hand, the signal light LS generated by the optical coupler 162 is guided to the end of the connection line 152 through an optical fiber 164 composed of a single mode fiber or the like. The conductive optical fiber 152a runs inside the connection line 152. Here, the optical fiber 164 and the optical fiber 152a may be composed of one optical fiber, or may be integrally formed by connecting the end faces of the respective fibers. In either case, it is sufficient as far as the optical fiber 164 and 152a are configured to be capable of transferring the signal light LS between the retinal camera unit 1 A and the OCT unit 150.

The signal light LS is guided through the inside of the connection line 152 and led to the retinal camera unit 1 A. Furthermore, the signal light LS is projected to the eye E through the lens 142, the scan unit 141, the dichroic mirror 134, the imaging lens 126, the relay lens 125, the variable magnifying lens 124, the imaging diaphragm 121, the aperture 112a of the aperture mirror 112 and the objective lens 113. The barrier filter 122 and 123 are retracted from the optical path in advance, respectively, when the signal light LS is projected to the eye E.

The signal light LS having entered into the eye E forms an image on the fundus oculi Ef and is then reflected. At this moment, the signal light LS is not only reflected on the surface of the fundus oculi Ef, but also scattered at the refractive index boundary after reaching the deep area of the fundus oculi Ef. Therefore, the signal light LS having passed through the fundus oculi Ef contains information reflecting the morphology of the surface of the fundus oculi Ef and information reflecting the state of backscatter at the refractive index boundary of the deep area tissue of the fundus oculi Ef. This light may be simply referred to as the "fundus oculi reflection light of the signal light LS."

The fundus oculi reflection light of the signal light LS travels reversely along the abovementioned path within the retinal camera unit 1A, and is converged to the end face 152b of the optical fiber 152a. The, the signal light LS enters into the OCT unit 150 through the optical fiber 152a, and returns to the optical coupler 162 through the optical fiber 164.

The optical coupler 162 superimposes the signal light LS having returned through the eye E and the reference light LR reflected by the reference mirror 174, thereby generating the interference light LC. The generated interference light LC is guided into a spectrometer 180 through an optical fiber 165 composed of a single mode fiber or the like.

Although a Michelson-type interferometer is adopted in this embodiment, it is possible to properly employ, for instance, a Mach Zender type, etc. and any type of interferometer.

The interference-light generator" of the present invention comprises, for example, an optical coupler 162, an optical member on the optical path of the signal light LS (i.e., an optical member placed between the optical coupler 162 and the eye E), and an optical member on the optical path of the reference light LR (i.e., an optical member placed between the optical coupler 162 and the reference mirror 174), and specifically, comprises an interferometer equipped with the optical coupler 162, the optical fibers 163 and 164, and the reference mirror 174.

The spectrometer 180 comprises a collimator lens 181, a diffraction grating 182, an image-forming lens 183, and a CCD 184. The diffraction grating 182 may be a transmission-type diffraction grating that transmits light, or may be a reflection-type diffraction grating that reflects light. Moreover, it is also possible to use, instead of the CCD 184, another photodetecting element such as a CMOS .

The interference light LC having entered into the spectrometer 180 is split (resolved into spectra) by the diffraction grating 182 after converged to a parallel light flux by the collimator lens 181. The split interference light LC is formed into an image on the image pick-up face of the CCD 184 by the image-forming lens 183. The CCD 184 detects the respective spectra of the split interference light LC and converts to electrical detection signals, and outputs the detection signals to the arithmetic and control unit 200. The CCD 184 is an example of the "detector" of the present invention.

### [Configuration of Arithmetic and Control Unit]

Next, the configuration of the arithmetic and control unit 200 will be described. The arithmetic and control unit 200 analyzes detection signals inputted from the CCD 184 of the OCT unit 150, and forms a tomographic image of the fundus oculi Ef. The analysis method here is the same as the conventional Fourier Domain OCT method.

Further, the arithmetic and control unit 200 forms a 2-dimensional image showing the morphology of the surface of the fundus oculi Ef, based on the video signals outputted from the imaging devices 10 and 12 of the retinal camera unit 1 A.

Furthermore, the arithmetic and control unit 200 controls each part of the retinal camera unit 1 A and the OCT unit 150.

As control of the retinal camera unit 1 A, the arithmetic and control unit 200 executes, for example: control of output of the illumination light by the observation light source 101 or the imaging light source 103; control of insertion/retraction operations of the exciter filters 105 and 106 or the barrier filters 122 and 123 to/from the optical path; control of the operation of a display device such as the LCD 140; control of movement of the illumination diaphragm 110 (control of the diaphragm value); control of the diaphragm value of the imaging diaphragm 121; and control of movement of the variable magnifying lens 124 (control of the magnification). Moreover, the arithmetic and control unit 200 executes control of the operation of the Galvano mirrors 141 A and 141B.

Further, as control of the OCT unit 150, the arithmetic and control unit 200 executes, for example: control of output of the low-coherence light L0 by the low-coherence light source 160; control of movement of the reference mirror 174; control of the rotary operation of the density filter 173 (the operation of changing the reduction amount of the reference light LR); and control of the accumulation time of the CCD 184.

The hardware configuration of the arithmetic and control unit 200 will be described referring to Fig. 4.

The arithmetic and control unit 200 is provided with a similar hardware configuration to that of a conventional computer. To be specific, the arithmetic and control unit 200 comprises: a microprocessor 201, a RAM202, a ROM203, a hard disk drive (HDD) 204, a keyboard 205, a mouse 206, a display 207, an image-forming board 208, and a communication interface (I/F) 209. These parts are connected via a bus 200a.

The microprocessor 201 includes a CPU (Central Processing Unit), an MPU (Micro Processing Unit) or the like. The microprocessor 201 executes operations characteristic to this embodiment, by loading a control program 204a stored in the hard disk drive 204, onto the RAM 202.

Further, the microprocessor 201 executes control of each part of the device described above, various arithmetic processes, etc. Moreover, the microprocessor 201 receives an operation signal from the keyboard 205 or the mouse 206, and executes control of each part of the device in response to the operation content. Furthermore, the microprocessor 201 executes control of a display process by the display 207, control of a transmission/reception process of data and signals by the communication interface 209.

The keyboard 205, the mouse 206 and the display 207 are used as user interfaces in the fundus oculi observation device 1. The keyboard 205 is used as, for example, a device for typing letters, figures, etc. The mouse 206 is used as a device for performing various input operations to the display screen of the display 207.

Further, the display 207 is a display device such as an LCD and a CRT (Cathode Ray Tube) display, and displays various images like the fundus oculi Ef formed by the fundus oculi observation device 1, or displays various screens such as an operation screen and a set-up screen.

The user interface of the fundus oculi observation device 1 is not limited to the above configuration, and may include a track ball, a control lever, a touch panel type of LCD, a control panel for ophthalmology examinations, etc. As a user interface, it is possible to employ any configuration having a function of displaying and outputting information and a function of inputting information and operating the device.

The image-forming board 208 is a dedicated electronic circuit for forming (image data of) images of the fundus oculi Ef. The image-forming board 208 is provided with a fundus oculi image forming board 208a and an OCT image forming board 208b.

The fundus oculi image forming board 208a is a dedicated electronic circuit that forms image data of fundus oculi images based on the video signals from the imaging device 10 and the imaging device 12.

Further, the OCT image forming board 208b is a dedicated electronic circuit that forms image data of tomographic images of the fundus oculi Ef, based on the detection signals from the CCD 184 of the OCT unit 150.

By providing the image-forming board 208, it is possible to increase the processing speed of a process for forming fundus oculi images and tomographic images.

The communication interface 209 sends control signals from the microprocessor 201, to the retinal camera unit 1A or the OCT unit 150. Moreover, the communication interface 209 receives video signals from the imaging devices 10 and 12 or detection signals from the CCD 184 of the OCT unit 150, and inputs the signals to the image-forming board 208. At this moment, the communication interface 209 operates to input the video signals from the imaging devices 10 and 12, to the fundus oculi image forming board 208a, and input the detection signals from the CCD 184, to the OCT image forming board 208b.

Further, in a case where the arithmetic and control unit 200 is connected to a communication network such as a LAN (Local Area Network) and the Internet, it is possible to configure to be capable of data communication via the communication network, by providing the communication interface 209 with a network adapter like a LAN card or communication equipment like a modem. In this case, by mounting a server accommodating the control program 204a on the communication network, and at the same time, configuring the arithmetic and control unit 200 as a client terminal of the server, it is possible to operate the fundus oculi observation device 1.

### [Configuration of Control System]

Next, the configuration of the control system of the fundus oculi observation device 1 will be described referring to Fig. 5.

### (Controller)

The control system of the fundus oculi observation device 1 is configured mainly having a controller 210 of the arithmetic and control unit 200. The controller 210 includes the microprocessor 201, the RAM202, the ROM203, the hard disk drive 204 (the control program 204a), and the communication interface 209.

The controller 210 executes the aforementioned control with the microprocessor 201 operating based on the control program 204a. The controller 210 is an example of the "controller" of the present invention.

Specifically, the controller 210 controls the mirror drive mechanisms 241 and 242 to regulate the positions of the Galvano mirrors 141 A and 141B, thereby scanning with the signal light LS so as to guide the viewpoint of the eye E in a specific direction. Here, the specific direction is, for example, as previously described, a direction in which the viewpoint of the eye E is moved in order to acquire the fundus oculi image Ef' or tomographic image in a target region of the fundus oculi Ef.

Further, the controller 210 executes control of the low-coherence light source 160 and the CCD 184, control of the density filter drive mechanism 244 for rotating the density filter 173, control of the reference-mirror drive mechanism 243 for moving the reference mirror 174 in the traveling direction of the reference light LR, etc.

Further, the controller 210 causes the display 240A of the user interface (UI) 240 to display two kinds of images captured by the fundus oculi observation device 1: that is, the fundus oculi image Ef' and a tomographic image. These images may be displayed on the display 240A separately, or may be displayed side by side.

### (Image forming part)

An image forming part 220 forms image data of the fundus oculi image Ef' based on the video signals from the imaging devices 10 and 12. Moreover, the image forming part 220 forms image data of the tomographic images of the fundus oculi Ef based on the detection signals from the CCD 184 of the OCT unit 150.

The imaging forming part 220 comprises the image-forming board 208 and the communication interface 209. In this specification, "image" may be identified with "image data" corresponding thereto.

### (Image Processor)

The image processor 230 applies various image processing and analysis processes to image data of images formed by the image forming part 220. For example, the image processor 230 executes various correction processes such as brightness correction and dispersion compensation of the images.

Further, the image processor 230 applies an interpolation process of interpolating pixels between tomographic images formed by the image forming part 220 to the tomographic images, thereby forming image data of a 3-dimensional image of the fundus oculi Ef.

Herein, image data of a 3-dimensional image is image data made by assigning pixel values to each of a plurality of voxels arranged 3-dimensionally, and is referred to as volume data, voxel data, or the like. In the case of displaying an image based on volume data, the image processor 230 applies a rendering process (such as volume rendering and MIP (Maximum Intensity Projection)) to this volume data, and forms image data of a pseudo 3-dimensional image seen from a specific view direction. On a display device such as the display 207, the pseudo 3-dimensional image based on the image data is displayed.

Further, the image processor 230 is also capable of forming stack data of a plurality of tomographic images. Stack data is image data that can be obtained by arranging a plurality of tomographic images acquired along a plurality of scanning lines based on the positional relationship of the scanning lines.

The image processor 230 operating as described above comprises the microprocessor 201, the RAM 202, the ROM 203, the hard disk drive 204 (control program 204a), etc.

### (User Interface)

The user interface (UI) 240 is provided with the display 240A and an operation part 240B. The display 240A is composed of a display device such as the display 207. The operation part 240B is composed of an input device or operation device such as the keyboard 205 and the mouse 206.

### [Signal Light Scanning and Image Processing]

Scan with the signal light LS is performed by turning around the reflecting surfaces of the Galvano mirrors 141A and 141B of the scan unit 141 as described before. The controller 210 controls the mirror drive mechanisms 241 and 242, respectively, to turn around the reflecting surfaces of the Galvano mirrors 141 A and 141 B, respectively, thereby scanning the fundus oculi Ef with the signal light LS.

When the reflecting surface of the Galvano mirror 141A is turned around, scan with the signal light LS in the horizontal direction (the x-direction in Fig. 1) is performed on the fundus oculi Ef. On the other hand, when the reflecting surface of the Galvano mirror 141 B is turned around, scan with the signal light LS in the vertical direction (the y-direction in Fig. 1) is performed on the fundus oculi Ef. Further, by turning around both the reflecting surfaces of the Galvano mirrors 141A and 141B simultaneously, it is possible to scan with the signal light LS in the composed direction of the x-direction and y-direction. That is, by controlling these two Galvano mirrors 141A and 141B, it is possible to scan with the signal light LS in any direction on the x-y plane.

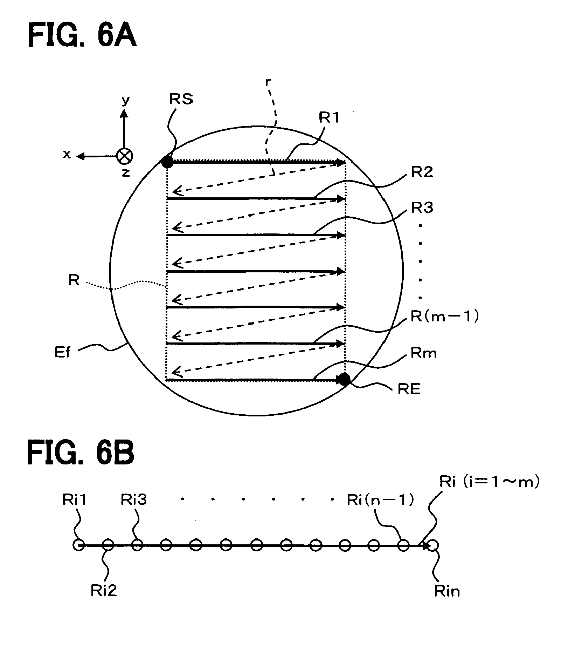

Figs. 6A and 6B show an example of a scanning pattern of the signal light LS for forming an image of the fundus oculi Ef. Fig. 6A shows an example of the scanning pattern of the signal light LS when the fundus oculi Ef is seen from a direction in which the signal light LS enters the eye E (that is, seen from the -z side to the +z side in Fig. 1). Further, Fig. 6B shows an example of an arrangement pattern of scanning points (positions to perform image measurement) on each scanning line on the fundus oculi Ef.

As shown in Fig. 6A, scan with the signal light LS is performed within a rectangular scanning region R set in advance. Within the scanning region R, a plurality of (m number of) scanning lines R1 to Rm are set in the x-direction. When scan with the signal light LS is performed along each scanning line Ri (i=1 to m), a detection signal of the interference light LC is generated.

A direction of each scanning line Ri will be referred to as the "main scanning direction," and a direction orthogonal thereto will be referred to as the "sub-scanning direction." Accordingly, scan with the signal light LS in the main scanning direction is performed by turning around the reflecting surface of the Galvano mirror 141A. Scan in the sub-scanning direction is performed by turning around the reflecting surface of the Galvano mirror 141 B.

On each scanning line Ri, as shown in Fig. 6B, a plurality of (n number of) scanning points Ri1 to Rin are set in advance.

In order to execute the scan shown in Figs. 6A and 6B, the controller 210 firstly controls the Galvano mirrors 141A and 141 B to set the entering target of the signal light LS into the fundus oculi Ef to a scan start position RS (a scanning point R11) on a first scanning line R1. Subsequently, the controller 210 controls the low-coherence light source 160 to flush the low-coherence light L0, thereby making the signal light LS enter the scan start position RS. The CCD 184 receives the interference light LC based on the fundus oculi reflection light of this signal light LS at the scan start position RS, and outputs the detection signal to the controller 210.

Next, the controller 210 controls the Galvano mirror 141A to scan with the signal light LS in the main scanning direction and set the entering target thereof to a scanning point R12, and causes the low-coherence light LO to flush to make the signal light LS enter a scanning point R12. The CCD 184 receives the interference light LC based on the fundus oculi reflection light of this signal light LS at the scanning point R12, and outputs the detection signal to the controller 210.

In the same way, the controller 210 makes the low-coherence light L0 flush at each scanning point while moving the entering target of the signal light LS from a scanning point R13 to R14, ----, R1 (n-1) and R 1 n in order, thereby obtaining a detection signal outputted from the CCD 184 in response to the interference light LC for each scanning point.

When the measurement at the last scanning point R 1 n of the first scanning line R1 ends, the controller 210 controls the Galvano mirrors 141A and 141 B simultaneously to move the entering target of the signal light LS to a first scanning point R21 of a second scanning line R2 along a line switching scan r. Then, by conducting the aforementioned measurement for each scanning point R2j (j=l to n) of this second scanning line R2, a detection signal corresponding to each scanning point R2j is obtained.

In the same way, the measurement is performed for each of a third scanning line R3, ----, an m-lth scanning line R(m-1) and an mth scanning line Rm, whereby a detection signal corresponding to each scanning point is acquired. Symbol RE on the scanning line Rm is a scan end position corresponding to a scanning point Rmn.

As a result, the controller 210 obtains m x n number of detection signals corresponding to m x n number of scanning points Rij (i=l to m, j=1 to n) within the scanning region R. Hereinafter, a detection signal corresponding to the scanning point Rij may be represented by Dij.

Interlocking control of movement of the scanning point and emission of the low-coherence light LO as described above can be realized by synchronizing transmission timing of control signals to the mirror drive mechanisms 241 and 242 with transmission timing of a control signal to the low-coherence light source 160.

As described above, when causing each of the Galvano mirrors 141A and 141 B to operate, the controller 210 stores the position of the scanning line Ri and the position of the scanning point Rij (coordinates on the x-y coordinate system) as information representing the content of the operation. This stored content (the scan position information) is used in an image forming process as conventional.

Next, an example of image processing in the case of scan with the signal light LS shown in Figs. 6A and 6B will be described.

The image forming part 220 forms tomographic images of the fundus oculi Ef along each scanning line Ri (the main scanning direction). Further, the image processor 230 forms a 3-dimensional image of the fundus oculi Ef based on the tomographic images formed by the image forming part 220.

A process for forming tomographic images by the image forming part 220 includes a 2-step arithmetic process as conventional. In the first step of the arithmetic process, based on the detection signal Dij corresponding to each scanning point Rij, an image in the depth-wise direction (the z-direction in Fig. 1) of the fundus oculi Ef at the scanning point Rij is formed.

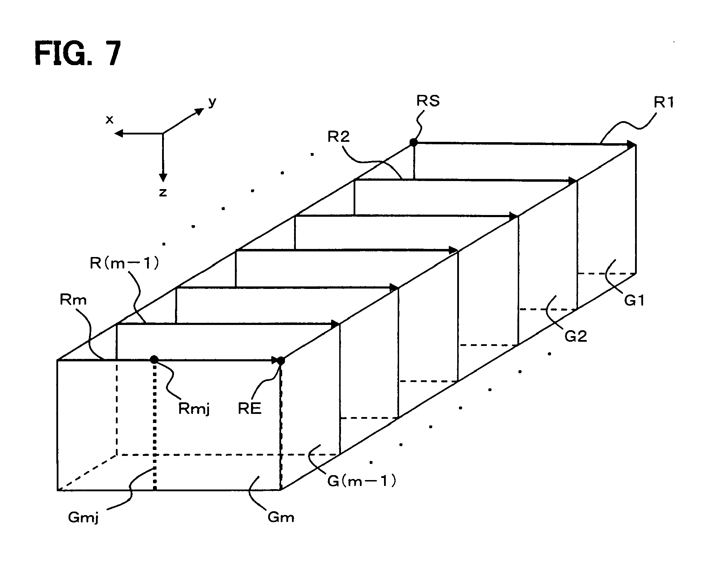

Fig. 7 shows a pattern of tomographic images formed by the image forming part 220. In the second step of the arithmetic process, for each scanning line Ri, based on the depth-wise images at the n number of scanning points Ri1 to Rin, a tomographic image Gi of the fundus oculi Ef along the scanning line Ri is formed. At this moment, the image forming part 220 determines the arrangement and interval of the scanning points Ri1 to Rin by referring to the positional information (scan position information described before) of the scanning points Ri1 to Rin, and forms this scanning line Ri. Through the above process, it is possible to obtain m number of tomographic images G1 to Gm at different positions in the sub-scanning direction (y-direction).

Next, a process for forming a 3-dimensional image of the fundus oculi Ef by the image processor 230 will be explained. A 3-dimensional image of the fundus oculi Ef is formed based on the m number of tomographic images obtained through the abovementioned arithmetic process. The image processor 230 forms a 3-dimensional image of the fundus oculi Ef, for example, by performing a known interpolating process of interpolating an image between the adjacent tomographic images Gi and G (i +I).

At this moment, the image processor 230 determines the arrangement and interval of the scanning lines Ri by referring to the positional information of the scanning lines Ri, thereby forming a 3-dimensional image. For this 3-dimensional image, 3-dimensional coordinates (x,y,z) are set based on the positional information of each scanning point Rij (the aforementioned scan position information) and the z coordinate in a depth-wise image.

Further, based on this 3-dimensional image, the image processor 230 can form a tomographic image of the fundus oculi Ef at a cross section in any direction other than the main scanning direction (x-direction). When the cross section is designated, the image processor 230 specifies the position of each scanning point (and/or an interpolated depth-wise image) on this designated cross section, extracts a depth-wise image at each of the specified positions (and/or an interpolated depth-wise image) from the 3-dimensional image, and arranges the plurality of extracted depth-wise images, thereby forming a tomographic image of the fundus oculi Ef at the designated cross section.

An image Gmj shown in Fig. 7 represents an image in the depth-wise direction (z-direction) at the scanning point Rmj on the scanning line Rm. In the same way, a depth-wise image at each scanning point Rij on the scanning line Ri formed in the aforementioned first-step arithmetic process is represented as the "image Gij."

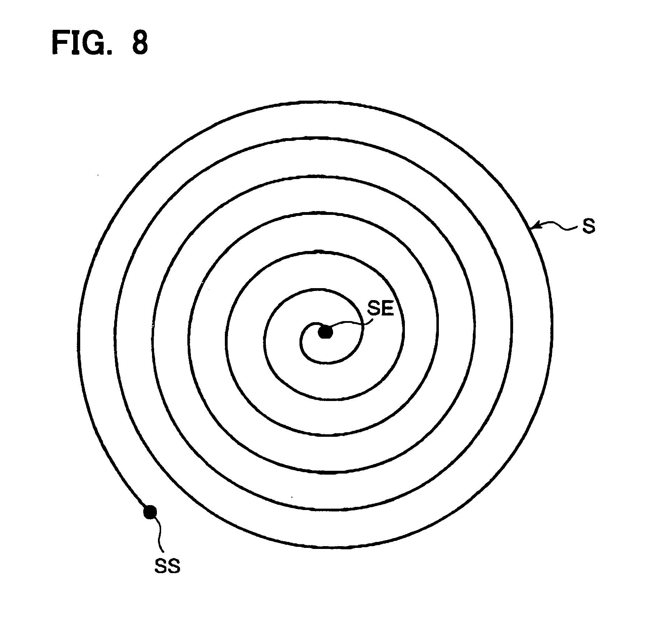

Next, the aforementioned scan with the signal light LS for guiding the viewpoint of the eye E in a specific direction will be described. A spiral scanning line S shown in Fig. 8 represents the trajectory of the scan. As in Fig. 6, a plurality of scanning points are previously set on the scanning line S. The number of the scanning points (namely, the interval between the adjacent scanning points) is previously set by, for example, an operator.

The controller 210 makes the signal light LS applied onto each scanning point, thereby executing this spiral scan. In the present embodiment, the scan starts from a scan start position SS that is outermost on the scanning line S. Furthermore, the controller 210 performs the scan while rotating the application position of the signal light LS toward the inside along the spiral scanning line S. Then, the controller 210 ends the scan at a scan end position SE that is innermost (the central position of the spiral) on the scanning line S.

The scan end position SE is set in the abovementioned specific direction, that is, in a direction to which the viewpoint of the eye E is turned in order to acquire an image in a target region of the fundus oculi Ef.

### [USAGE PATTERN]

A usage pattern of the fundus oculi observation device 1 will be described. Two usage patterns of the fundus oculi observation device 1 will be described below. A first usage pattern is a usage pattern in the case of scan with the signal light LS along a spiral trajectory. A second usage pattern is a usage pattern in the case of scan with the signal light LS in the main scanning direction and the sub-scanning direction. In the second usage pattern, the aforementioned visible information is presented.

### [Usage Pattern 1]

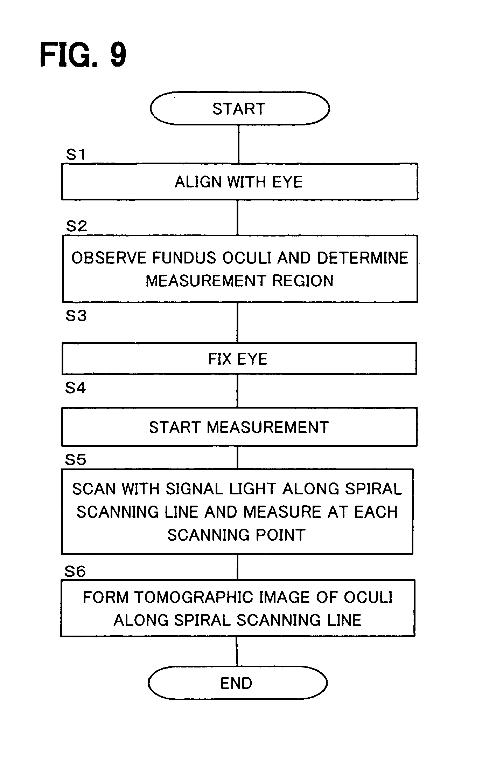

A flowchart of Fig. 9 shows an example of a usage pattern in the case of scan with the signal light LS along a spiral trajectory.

First, the eye E is positioned at a specific measurement position (a position facing the objective lens 113), and the optical systems 100 and 120 of the retinal camera unit 1A are aligned with the eye E (S1).

When the alignment is completed, the operator observes the fundus oculi Ef with the retinal camera unit 1A, and determines a measurement region of the fundus oculi Ef (S2). When the operator sets a fixation position by operating the operation part 240B, the controller 210 controls the LCD 140 to display an internal fixation target corresponding to the set fixation position (S3). When the eye E is fixed, display of the internal fixation target is ended.

When the operator request to start measurement through the operation part 240B (S4), the controller 210 controls to scan with the signal light LS along the spiral scanning line S shown in Fig. 8. Consequently, a detection signal corresponding to each scanning point on the scanning line S can be acquired (S5). Each detection signal is inputted into the image forming part 220 from the CCD 184.

The image forming part 220 forms a tomographic image of the fundus oculi Ef along the spiral scanning line S based on the detection signals from the CCD 184 (S6). This process can be executed as in Fig. 7.

As necessary, the image processor 230 forms a 3-dimensional image, or forms a tomographic image at any cross-sectional position. This is the end of description of the usage pattern.

### [Usage Pattern 2]

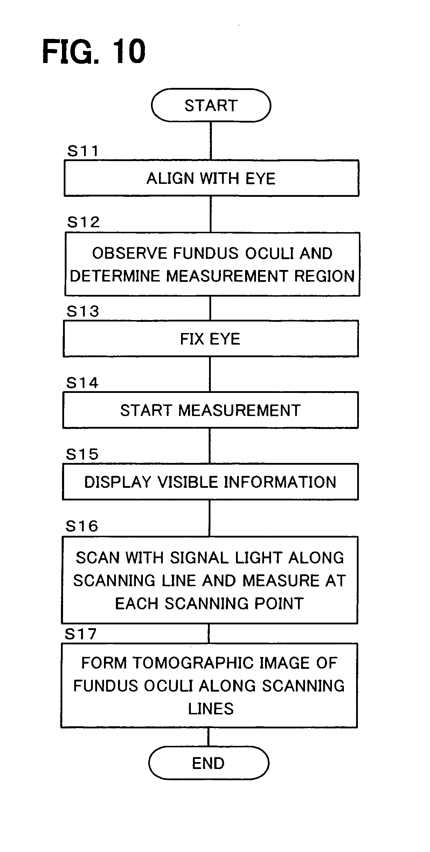

A flowchart of Fig. 10 shows an example of a usage pattern in a case where visible information is presented while scan with the signal light LS is performed in the main scanning direction and sub-scanning direction. In a case where visible information is presented, a scanning pattern of the signal light LS is arbitrary.

First, as in the first embodiment, the eye E is positioned at a specific measurement position and the alignment is performed (S11), the operator determines the measurement region of the fundus oculi Ef (S 12), and the eye E is fixed (S 13). The display of the internal fixation target is ended when the eye E is fixed.

When the operator requests to start measurement (S14), the controller 210 controls the LCD 140 to display the visible information (S15) and performs scan with the signal light LS along the scanning lines R1 to Rm, thereby acquiring detected signals corresponding to the respective scanning points Rij (S 16). The detection signals are inputted into the image forming part 220 from the CCD 184.

The image forming part 220 forms the tomographic images Gi of the fundus oculi Ef along the respective scanning lines Ri based on the detection signals from the CCD 184 (S 17).

As necessary, the image processor 230 forms a 3-dimensional image, or forms a tomographic image at any cross-sectional position. This is the end of description of the usage pattern.

### [ACTIONS AND ADVANTAGEOUS EFFECTS]

Actions and advantageous effects of the fundus oculi observation device 1 as described above will be described below.

The fundus oculi observation device 1 functions as an optical image measurement device configured to scan an eye with a light beam and form an image of the eye by using the reflected light. To be specific, the fundus oculi observation device 1 comprises: a function of generating the interference light LC by splitting the low-coherence light LO into the signal light LS and the reference light LR and superimposing the signal light LS having passed through the eye E and the reference light LR having passed through the reference mirror 174 (i.e., the interference-light generator); a function of detecting the interference light LC (i.e., the detector); and a function of scanning an application position (projection position) of the signal light LS on the eye E (i.e., the scanner), and the fundus oculi observation device 1 forms a tomographic image or 3-dimensional image of the fundus oculi Ef of the eye E based on the detection result of the interference light LC.

Further, the fundus oculi observation device 1 has a function of controlling the scanner to scan with the signal light LS so as to guide the viewpoint of the eye E to a specific direction (i.e., the controller). In the present embodiment, it is possible to guide the viewpoint of the eye E to the spiral center SE, by scanning with the signal light LS along the spiral trajectory (the scanning line S). Here, the position of the spiral center SE is determined in accordance with the measurement region of the fundus oculi Ef.

By thus guiding the viewpoint of the eye E, it is possible to prevent the eye E from following the scan trajectory, and acquire an accurate image. The trajectory viewed at the time of such scan acts to fix the eye E, and is an example of the "fixation information" in the present invention.

Furthermore, the fundus oculi observation device 1 can use different type of fixation information from the abovementioned one. That is, it is possible to project visible information other than the scan trajectory of the signal light LS, to the eye E as the fixation information.

As previously described, there are various types of visible information. These include: (1) visible information presented brighter than the trajectory of the signal light LS; (2) visible information presented in a different color from that of the trajectory of the signal light LS; (3) static visible information that acts to make the eye E fix on one point; (4) dynamic visible information whose form changes independently from the trajectory of the signal light LS; and (5) dynamic visible information whose form changes accompanying the trajectory of the signal light LS.

In the examples (4) and (5), the projection position of the visible information onto the fundus oculi Ef is moved, and specifically acts to guide the viewpoint of the eye E to a specific direction. Further, as previously described in the example (5), the visible information is moved in a direction heading toward the visual field center of the eye E at least.

The visible information is displayed on the LCD 140 (display). The displayed visible information passes through the lens 139, half mirror 135, field lens 128, dichroic mirror 136, imaging lens 126, relay lens 125, variable magnifying lens 124, (the aperture 112a of) the aperture mirror 112 and objective lens 113, and enters the eye E to be projected onto the fundus oculi Ef. These optical elements for projecting the visible information onto the fundus oculi Ef act as an example of the "projection optical system" in the present invention.

By projecting such visible information onto the fundus oculi Ef, it is possible to prevent the eye E from following a scan trajectory and fix the eye E, thereby acquiring an accurate image.

### [MODIFICATION]

The configuration described above is merely an example for favorably implementing the present invention. Therefore, it is possible to properly make any modification within the scope and intent of the present invention.

For example, as the projector for projecting the visible information onto the eye, it is possible to use a configuration including a light source and a projection optical system. The light source outputs a light used as the visible information. As the light source, it is possible to use any light source such as an LED (Light Emitting Diode), a laser light source and a lamp. Further, it is possible to provide any number of (one or more) light sources. The projection optical system is an optical system for projecting the light outputted from the light source onto a fundus oculi.

A specific example of such a projector will be described. For example, the light source outputs a light for presenting a luminescent point that is brighter than the trajectory of the signal light LS to the eye E. Further, the light source outputs a light for presenting a luminescent point with a color different from the trajectory of the signal light LS, to the eye E. It is possible to prevent the eye E from following the scan trajectory and acquire an accurate image, by projecting such luminescent point onto the fundus oculi Ef as the visible information. The luminescent point may be spread to some extent.

As a second example, a plurality of light sources are arranged in a specific pattern. In an example of the arrangement pattern, it is possible to arrange like an array in the vertical direction and horizontal direction, for example. The plurality of light sources are switched on and off individually in response to control by the controller (the controller 210). The controller switches on one or more light sources among the plurality of light sources when necessary, and projects one or more luminescent points onto the fundus oculi. By projecting such visible information onto the fundus oculi Ef, it is possible to prevent the eye E from following the scan trajectory and acquire an accurate image.

In a third example, the light source is moved by a drive mechanism equipped with a motor or the like. It is particularly preferable to move them in a direction orthogonal to the propagating direction of the light. Furthermore, the number of the light sources is arbitrary. In a case where a plurality of light sources are provided, the light sources may be moved individually, or two or more may be moved together. The controller controls the drive mechanism to move the light sources. Thus, the projection position of the luminescent point on the fundus oculi Ef is changed. It is possible to prevent the eye E from following the scan trajectory and acquire an accurate image, by projecting such visible information.

Another example of the visible information will be described. This example is for making a scan trajectory less outstanding by adopting a color that is (approximately) identical to the scan trajectory of a signal light LS as the background color of the visual field of the eye E. For this, as the visible information, the projector projects background information composed of substantially identical color to the scan trajectory of the signal light LS.

Because the low-coherence light LO has a central wavelength in a near-infrared region and the visible component contained therein is of a long wavelength range (i.e., a wavelength region equivalent to the red color), the scan trajectory is viewed in red color. Therefore, red-color visible information is projected in this example.

The color (the wavelength distribution) of the visible information may be theoretically determined based on the wavelength distribution of the low-coherence light L0, or may be determined by actually measuring the wavelength distribution of the signal light LS outputted from the objective lens 113.

Moreover, the color of the background information does not need to be completely the same as the color of the scan trajectory (the color of the signal light LS), and a difference to an extent that the scan trajectory does not stand out in the background color may be permitted. A specific example of the projector for projecting the background information will be described below.

In a first specific example, it is possible to configure so as to display background information by a display and project the background information onto the fundus oculi Ef by a projection optical system. This display is composed of, for example, any display device such as an LCD 140 and an LCD 109.

In a case where the LCD 140 is used as the display, the LCD 140 displays an image of a specific background color in the entire screen (or in a specific region in the screen), for example. This image is projected onto the fundus oculi Ef via a similar path (projection optical system) to that of the aforementioned internal fixation target.

Further, in a case where LCD 109 is used as the display, the LCD 109 displays an image of a specific background color in the entire screen (or in a specific region in the screen). Moreover, a light of a visible light source (e.g., the observation light source 101) is applied from behind the LCD 109. Consequently, the image displayed in the LCD 109 is projected onto the fundus oculi Ef via the same path (projection optical system) as the previously described illumination light.

In a second specific example, it is possible to provide a light source that outputs a light having a specific background color and configure so as to project the light onto the fundus oculi Ef through a projection optical system. This light source may be configured to emit a light having the specific background color by itself, or may be configured to generate a light having the background color by using a filter.

Because projection of such background information onto the fundus oculi Ef at the time of scan with the signal light LS makes it difficult to view the scan trajectory of the signal light LS, it is possible to prevent the eye E from following the scan trajectory.

Because the patient may feel it too bright, or because miosis of the eye E may occur, it is desirable to prohibit projection of the background information when scan with the signal light LS is not performed. Further, it is also possible to prevent the miosis by administering a mydriatic drug.

Further, it is possible to project a fixation target such as an internal fixation target onto the fundus oculi Ef, together with the background information. The color of the fixation target is desirable to be a color that is different from the background color (that is, a color that is easy to view in the background color).

In the above embodiment, the eye E is fixed by using a fixation-target projector prior to the measurement of OCT images. However, by displaying the fixation information according to the present invention at the time of measurement, it is possible to omit fixation performed in advance. That is, by displaying the fixation information at the time of measurement, it is possible to fix the eye E during measurement, even if fixation is not performed in advance.

Moreover, by continuously presenting the fixation target used prior to the measurement of the OCT images also during measurement, it is also possible to use this fixation target as the fixation information.

In the embodiment described above, an optical path length difference between an optical path of the signal light LS and an optical path of the reference light LR is changed by changing the position of the reference mirror 174, but the method for changing the optical path length difference is not limited to this. For instance, it is possible to change the optical path length difference by integrally moving the retinal camera unit 1 A and the OCT unit 150 with respect to the eye E and changing the optical path length of the signal light LS. Further, it is also possible to change the optical path length difference by moving a measurement object in the depth direction (z-direction).

Although the fundus oculi observation device described in the above embodiment comprises an optical image measurement device of a Fourier-domain type, it is possible to apply, to the present invention, any optical image measurement device with a system such as a Swept Source type, in which an eye is scanned by a light beam.

Further, in the above embodiment, a device for acquiring OCT images of a fundus oculi is described. However, it is also possible to apply the configuration of the above embodiment to, for example, a device capable of acquiring OCT image of other locations of an eye such as the cornea.

## Claims

1. An optical image measurement device that has:
an interference-light generator configured to generate an interference light by splitting a low-coherence light into a signal light and a reference light and superimposing the signal light having passed through an eye and the reference light having passed through a reference object;
a detector configured to detect the generated interference light; and
a scanner configured to scan a projection position of the signal light on the eye, and that is configured to form an image of the eye based on a result of detection by the detector, the optical image measurement device comprising:
a projector configured to project fixation information for fixing the eye onto a fundus oculi of the eye when the scanner scans with the signal light.

2. The optical image measurement device according to Claim 1, wherein:
the projector includes a controller configured to control the scanner to scan with the signal light so as to guide a viewpoint of the eye to a specific direction.

3. The optical image measurement device according to Claim 1, wherein:
the controller controls to scan with the signal light along a spiral trajectory.

4. The optical image measurement device according to Claim 1, wherein:
the projector projects visible information other than the scan trajectory of the signal light as the fixation information.

5. The optical image measurement device according to Claim 4, wherein:
the projector moves a projection position of the visible information on the fundus oculi.

6. The optical image measurement device according to Claim 5, wherein:
the projector moves the projection position of the visible information so as to guide a direction of the eye to a specific direction.

7. The optical image measurement device according to Claim 5, wherein:
the projector moves the visible information at least in a direction heading to a visual field center of the eye.

8. The optical image measurement device according to Claim 4, wherein:
the projector projects background information of a substantially same color as the scan trajectory of the signal light, as the visible information.

9. The optical image measurement device according to Claim 4, wherein:
the projector includes a display configured to display the visible information and a projection optical system configured to project the displayed visible information onto the fundus oculi.

10. The optical image measurement device according to Claim 5, wherein:
the projector includes a display configured to display the visible information and a projection optical system configured to project the displayed visible information onto the fundus oculi.

11. The optical image measurement device according to Claim 6, wherein:
the projector includes a display configured to display the visible information and a projection optical system configured to project the displayed visible information onto the fundus oculi.

12. The optical image measurement device according to Claim 7, wherein:
the projector includes a display configured to display the visible information and a projection optical system configured to project the displayed visible information onto the fundus oculi.

13. The optical image measurement device according to Claim 8, wherein:
the projector includes a display configured to display the visible information and a projection optical system configured to project the displayed visible information onto the fundus oculi.

14. The optical image measurement device according to Claim 4, wherein:
the projector includes a light source, and a projection optical system configured to project a light outputted from the light source onto the fundus oculi, as the visible information.

15. The optical image measurement device according to Claim 5, wherein:
the projector includes a light source, and a projection optical system configured to project a light outputted from the light source onto the fundus oculi, as the visible information.

16. The optical image measurement device according to Claim 6, wherein:
the projector includes a light source, and a projection optical system configured to project a light outputted from the light source onto the fundus oculi, as the visible information.

17. The optical image measurement device according to Claim 7, wherein:
the projector includes a light source, and a projection optical system configured to project a light outputted from the light source onto the fundus oculi, as the visible information.

18. The optical image measurement device according to Claim 8, wherein:
the projector includes a light source, and a projection optical system configured to project a light outputted from the light source onto the fundus oculi, as the visible information.

19. The optical image measurement device according to Claim 2 further comprising:
a fixation-target projector configured to project a fixation target onto the eye before the scanner scans with the signal light,
wherein the projector projects the fixation information so as to guide the eye in a same direction as a projection position of the fixation target as the specific direction.

20. The optical image measurement device according to Claim 6 further comprising:
a fixation-target projector configured to project a fixation target onto the eye before the scanner scans with the signal light,
wherein the projector projects the fixation information so as to guide the eye in a same direction as a projection position of the fixation target as the specific direction.
